# EUROPEAN PATENT APPLICATION

(11) **EP 1 018 324 A1**
(43) Date of publication of application: **12.07.2000**
(21) Application number: 99100116.5
(22) Date of filing: 04.01.1999
(51) Int. Cl.: A61F 5/451

(54) **Container for the collection of bodily waste provided with a wrapper**

(71) Applicant: THE PROCTER & GAMBLE COMPANY, Cincinnati, Ohio 45202 (US)
(72) Inventor: D'Acchioli, Vincenzo, 65779 Kelkheim am Taunus (DE); Coles, Peter, 65830 Kriftel (DE); Evangelista, Olindo, 66023 Francavilla al Mare, Chieti (IT); Schöne, Rainer, 1462 Königstein/Ts (DE)
(74) Representative: Kohol, Sonia

(57) **Abstract**

The present invention relates to human waste management devices suitable for babies, children or adults. The invention resides principally in providing such devices with a convenient individual packaging, such as a wrapper (48), which comprises a resealable opening means.

## Description

### Field of the invention

The present invention relates to human waste management devices for babies, children or adults. Said devices are provided with a wrapping material for individual packaging.

### Background of the invention

Human waste management devices are known articles of manufacture that are designed to be worn principally by incontinence sufferers and in particular by bedridden patients. Such human waste management devices are attached to the perianal or uro-genital region of the wearer and are intended to entrap and immediately contain faecal material, urine and other bodily discharges. Such devices, as they are mostly known today are constituted of a bag at one extremity of which is positioned the aperture and the attachment device, which typically is adhesive.

For example faecal management devices are disclosed in e. g. the following documents: US 3,577,989, which details a disposable plastic bag for incontinence sufferers. US 4,784,656 describes a receptacle for collecting faecal matter. The receptacle is formed from two sheets of thermoplastic film that are heat sealed along their side edges. GB 2 152 387, which teaches a faecal collector for incontinence sufferers comprising a collection bag consisting of a pair of panels of thermoplastic sheet material joined at their margins.

EP 245 064 discloses bags substantially consisting of a front and a rear wall, which are made of a synthetic plastic material, such as PVC. GB 2 215 605 discloses an ostonomy bag comprising panels of synthetic plastic material, the rear bag wall further comprising a needled film.

Plastic sheets, as typically used for the faecal management devices of the mentioned prior art, will typically easily suffer damage from contact with sharp objects or chemicals. Such damage, while often not readily visible, may initiate rupture under strain during later usage. Rupture in use of the faecal management device is one of the most unwanted occurrences of misfunctioning of the device.

For attachment to a wearer, faecal management devices are most commonly provided with an adhesive layer on the flange. Such adhesive layers, in particular hydrocolloid layers as taught in EP 0 753 290, have a much reduced sticking force to the wearers skin when portions of that layer have previously been affected by dust, dirt or the like.

Urine management devices are, for instance, disclosed in the following documents: GB 1,092,274 discloses a pediatric urine collector for female use comprising a collector bag of plastic material opening. The base of the opening is provided with a wedge like projection adapted to engage the lower perineal area of the infant. The collector is secured to the body of the wear by adhesive material. GB 2,268,882 discloses a urostomy pouch/bag of plastic material provided with a circular stomal orifice which is surrounded by a first coupling member, by which the pouch can be affixed to a counterpart coupling member, which can be attached to a wearer. The pouch may also be provided for use as a kit further comprising an applicator of super absorbent material which can be injected into the pouch by the use of a plunger. US 4,804,377 discloses a collector for urine specimens from children. The collector comprises a rectangular flange for adhesive attachment, which comprises a round, slightly oval aperture. EP 140 478 discloses a disposable diaper having a water proof barrier preferably polypropylene or polyethylene formed as a flattened bag having a single opening. US 1,092,274 discloses a urine collector for female infants comprising an aperture which is smaller at the bottom end than at the top end and could be described as droplet shaped. US 3,292,626 also discloses a urine collector for female infants comprising a circular or droplet shaped aperture. Chinese patent application CN 1079381 discloses urine bags for infants with circular and elliptical apertures.

Reliable adhesive attachment is a problem known in the field of human waste management devices. With regard to the skin of the wearer, which suffers from an aggressive adhesive, the properties of the adhesive are to be carefully chosen. Deposition of dirt on such a carefully chosen adhesive could lead to unintentional detachment. Such unintentional detachment is known in the art, as mentioned in GB 2 116 849.

While the adhesive layer may be protected to some extend by the use of the release means, further protection is desirable.

All human waste management devices disclosed in the documents, which are referred to above, are typically used for bedridden patients, independent of whether they are attached to the natural or an artificial anus. However, in a recent patent application (EPO application 97110603.4; "Shaped faecal management device") and co-pending applications, faecal management devices are disclosed which are optimised also in respect to the use for babies. Such use encompasses that the human waste management device is stored, transported and carried in a large variety of circumstances. For example, a mother may want to carry one or more devices in her handbag. By mechanical contact with a multitude of other items not uncommonly carried in a handbag, the faecal management device may suffer damage, or, not less likely, dirt, dust or chemical substances may be deposited on parts of the faecal management device. This is naturally highly undesirable.

Colostomy bags are typically provided in individual packages which are completely sealed. Such packages are typically provided from two plastic sheets which are permanently sealed along four edges. Such individual packages however are not suitable for faecal management devices for use on babies, where the circumstances of use demand that the mother is able to easily, quickly and conveniently open and remove the device from the package.

Moreover, there is a demand to have of individual package for transport of the device after use, in particular when such are device is used by a mother on a baby when she travels etc.

It has now been found, that all of the above outlined problems can be addressed by providing a human waste management device in a convenient individual package, such as a wrapper comprising a resealable opening means.

### Summary of the invention

The present invention relates to human waste management devices for babies, children or adults. The invention resides principally in providing such devices with a convenient individual package, such as a wrapper (48), which is provided with a resealable opening means.

### Brief description of the drawings

It is believed that the invention will be better understood from the foregoing description in conjunction with the accompanying drawings in which:
Figure 1 is a perspective view of a preferred embodiment of the faecal management device.
Figure 2 is a perspective view of a wrapper for individual packaging of a faecal management device comprising an applicator. The package is shown in its closed configuration.
Figure 3 is a cross sectional view along line 3-3 of Figure 2.
Figure 4 is a perspective view of a of a wrapper for individual packaging of a faecal management device comprising an applicator. The package is shown in an opened configuration.
Figure 5 is a top plan view of a preferred embodiment of a urine management device. A folding pattern for individual packaging is indicated.

### Detailed description of the invention

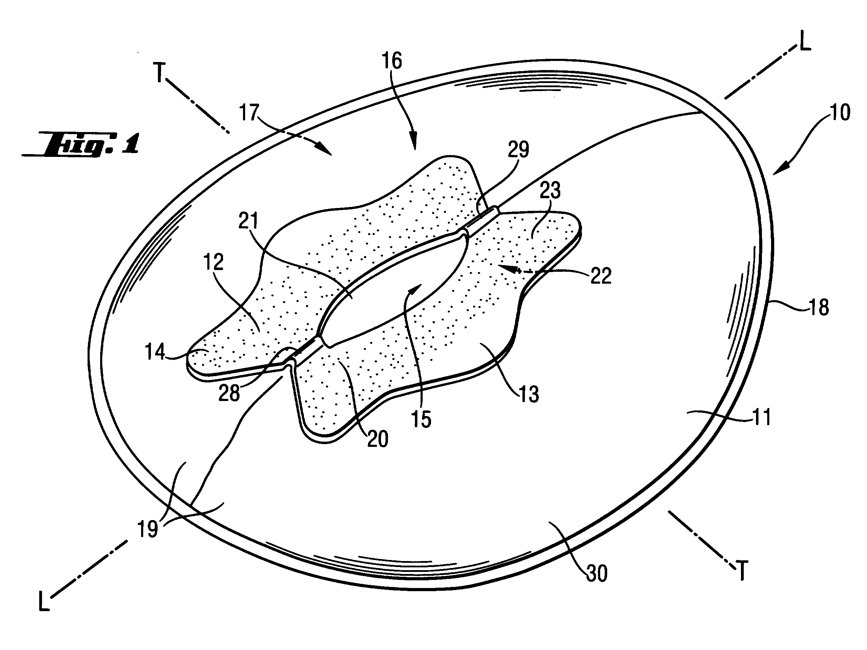

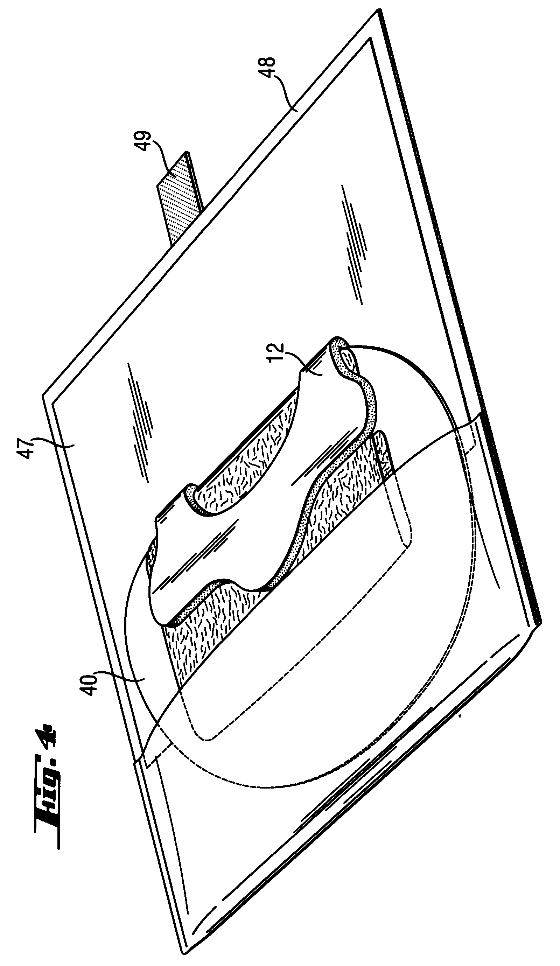

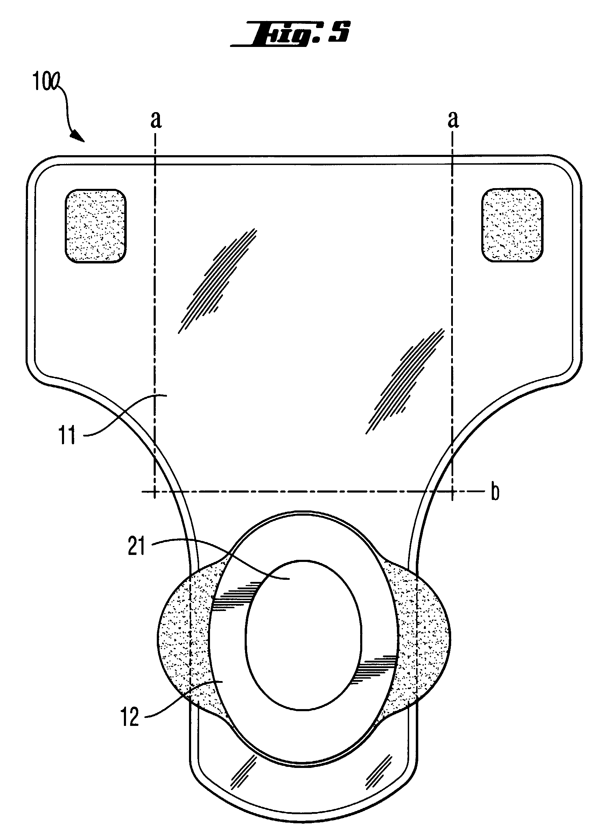

The invention relates to human waste management devices. Such a human waste management device may be a faecal management device (10), which is designed for attachment to the anal area and mainly used for collecting faeces, or it may be a urinary management device, which is attached to the urinary duct and mainly used for collecting urine. A human waste management device may also be a device to collect both urine and faeces and thus be attached to both of the above areas. All of the above human waste management devices are preferably designed for single use and disposal thereafter. A faecal management device (10) is shown in Figure 1. A urine management device (100) is shown in Figure 5.

### Description of the human waste management device as a whole

Typically human waste management devices comprise a bag (11) having an aperture (21) and a flange (12) surrounding the aperture for preferably adhesive attachment to the perianal area of a wearer as visible from Figure 1. Any human waste management device known in the art can be provided according to the present invention.

The bag (11) as used herein is a flexible receptacle for the containment of excreted faecal matter or urine. The bag (11) is designed to safely contain any entrapped material, typically it will be liquid impermeable, yet it may be breathable. The bag (11) is designed of sufficient strength to withstand rupture in use, also when pressure on the bag (11) is exerted in typical wearing conditions, such as sitting.

According to the present invention the bag material can comprise one or multiple layers, preferably two or three layers. The layer on the inside of the bag (11), which will typically at least partially come in contact with faecal material or urine is called the inner layer. The outermost layer of the bag, which will typically at least partially come in contact with the skin to the wearer and the garments of the wearer, is called the outer layer.

The layers of the bag material may comprise any material, preferably so that the bag is liquid impervious. The preferred optical properties of any such material will be described below in more detail. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag material may comprise a non-woven layer. Such material layers present an uneven surface to the skin of the wearer and thus reduce significantly the problem of occlusion and greatly improve skin healthiness.

In one preferred embodiment of the present invention the bag material comprises two layers. Preferably the outer layer comprises a non-woven layer and the inner layer comprises a film.

In yet another preferred embodiment of the present invention, the bag material comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the bag material may be hydrophobic or hydrophilic. If the bag material does not comprise a film layer, preferably at least one non-woven layer is hydrophobic. As a consequence, fluid penetration is resisted through the wearer facing portion (16) and the garment facing portion (17) of the human waste management device (10). If the bag material comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, non-anionic, cationic and non-cationic surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

According to the present invention, depending on the shape of the bag (11) required, the bag (11) may be provided from a unitary piece of material or a number of separate pieces of material, which may be identical or different and which are sealed at their respective peripheries. The preferred shape of the bag depends in particular on the intended use thereof, i.e. whether the device is intended for bedridden patients or active patients suffering from incontinence or requiring an artificial bowel or for infants.

The bags described herein preferably have a wearer facing portion (16) and a garment facing portion (17), which both comprise separate pieces of material. The wearer facing portion (16) and the garment facing portion (17) are sealed at the periphery of the bag (11), thus creating a bag peripheral rim (18). The wearer facing portion (16) and the garment facing portion (17) may each independently comprise more than one section of material. Preferably the garment facing portion (17) comprises only one section of material; most preferably also the wearer facing portion (16) comprises only one section of material.

The wearer facing portion (16), the garment facing portion (17) and the pieces of material comprised by either of these portions are secured to each other by means known to the man skilled in the art, such as adhesive, thermobonding or pressure bonding in order to provide the desired bag configuration. The rim (18), at which the wearer facing portion (16) and the garment facing portion (17) are sealed together, may be provided inside the bag (11) rather than outside the bag (11), thus being coextensive with the inner surface (15) of the bag (11) rather than with the outer surface (30) of the bag (11).

Hence a variety of shapes of the bag is within the scope of the present invention. Particularly, preferred shapes are flat circular type bags, cone shaped bags, truncated cone shaped bags and pyramidal or truncated pyramidal shaped bags and flat T shaped bags.

In one embodiment of the present invention the bag (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner surface (15) of the bag (11). Any known techniques for securing absorbent material to nonwoven and film substrates may be used to secure the absorbent material to the inner surface (15) of the bag. The absorbent material may also be arranged to have any desired shape or configuration (e.g., rectangular, oval, circular, etc.).

The bag (11) is provided with an aperture (21) whereby faecal matter or urine is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction or in both directions, e.g. the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering faecal matter or urine.

The flange may be provided in any size depending on the wearer group for which the device is intended. Similarly the flange may be provided in any shape and preferably has a symmetrical shape preferably comprising a plurality of lobes (13).

The flange comprises a garment facing portion (22) and a wearer facing portion (23). In an preferred embodiment these are two large, substantially flat surfaces, however, the flange (12) may also comprise projections, a front projection (28) and/or a rear projection (29), in case of a faecal management device (10) designed to fit the perineal and/or coccygeal area of the wearer and in case of a urine management device (10) designed to fit the genital and/or perineal area.

The flange (12) should be made of soft, flexible and malleable material to allow easy placement of the flange (12) to the perianal or uro-genital area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/m², more preferably 50 g/m². Other thermoplastic foam materials, or other suitable plastics sheet materials having the described properties of such foams (i.e., softness, pliability, stretchability, and contractability) might also be used. Preferably, the material of garment facing portion (22) of the flange (12) may extend into the defined aperture area so as to form a skirt or flap of material which prevents unintentional adhesion of the surface edges of the flange (12) defining the aperture (21) to one another during use.

According to the present invention the human waste management device (10) further comprises an attachment means to secure the device to the wearer. Such means include straps and more preferably comprises a body-compatible pressure sensitive adhesive (20) applied to the wearer facing portion (23) of the flange (12).

The adhesive (20) is preferably covered with a release means (not shown) in order to protect the adhesive (20), such as siliconized paper. The adhesive (20) can cover the entire wearer facing portion (23) of the flange (12), more preferably the flange (12) has at least one, preferably two to six non-adhesive portions. These portions may be adhesive free or may contain inactivated or covered adhesives. As is evident from Figure 1, the adhesive is in one preferred embodiment not applied to the entire wearer facing portion (23) of the flange (12), so as to provide lobes (13) on either side of the flange (12) which are non-adhesive and can thereby serve to facilitate placement and removal of the device whilst avoiding contact with the adhesive. These lobes (13) are however preferably also covered by the release means. Before application of the human waste management device (10) to the skin of the wearer, the release means if present is removed.

According to the present invention any medically approved water resistant pressure sensitive adhesive may be used to attach the device to the perianal or uro-genital area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive (20) can be applied to the wearer facing portion (23) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example, for human waste management devices (10) to be used for babies the amount of adhesive (20) may be less than for human waste management devices (10) designed for active adult incontinence sufferers.

The human waste management device (10) of the present invention has been found to be particularly useful and beneficial when used in conjunction with a garment, or diaper (50), preferably a disposable diaper.

### Detailed description

According to the present invention the human waste management device is provided with an individual package. Such a human waste management device may be a faecal management device (10) or it may be a urinary management device. All references to urinary management devices as made herein refer to devices which comprise a storage means such as a bag and also to devices which do not have any storage capacity and typically serve to close the urethra.

The faecal management device (10) or urinary management device may be provided in combination with an applicator (40) or any other tool or aide for application, usage period and detachment. An individual package within the scope of the present invention may comprise any such an aide or tool, may it be for single or for multiple use. The individual package may also comprise both, a faecal management device (10) and an urinary management device.

The individual package may be provided from a single piece of material or from a multitude of pieces of material. Some of the comprised materials may be joined together. Preferably the individual package is provided from a wrapper (48). A wrapper (48) consisting of joint pieces of material is referred to as a one-part wrapper.

The individual package may be provided from any suitable material, such as plastic films, paper, cloth, non-wovens. It may comprise a multitude of layers of such materials. It may also be provided of the laminate comprising a multitude of layers of any of the materials listed above. A preferred material is a single layer of polyethylene foil.

The individual package may be provided from a transparent or from an opaque material or from a combination thereof. It may be provided with a printed or otherwise applied design or with usage instructions.

An individually packaged device is normally perceived as being aesthetically more pleasant then an unpackaged device and offers some discretion when transported, which is often appreciated considering the use of the device. An opaque package or a package with applied design is beneficial to that effect.

The individual package may comprise a release means for contact with adhesive portions of the faecal management device or urinary management device or any tool packaged therewith. Such release means may be provided as a separate piece of material which may or may not be joined to other parts of the individual package. Portions of the individual package may also be covered with a coating such as a silicone coating.

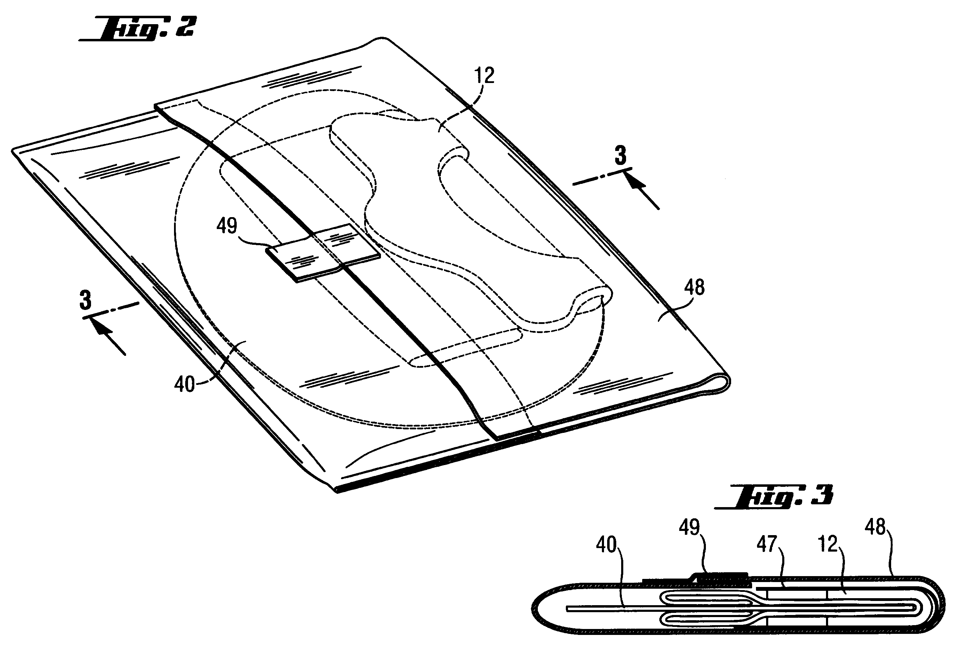

The individual package according to the present invention is provided with a means for opening and resealing the package. Such means may comprise adhesive, a hook/loop ensemble, such as Velcro®, a zipping mechanism, a button, an elastic, a string and other techniques known in the art. One preferred opening means comprises a tab (49) as shown in Fig. 2 to 4 which is provided with an adhesive for releasable and resealable adhesive attachment.

Any means referred to as opening means should allow easy opening by the use of a users fingers without the need to use any tool, such as scissors or a knife. A opening means is described herein as resealable if it allows attachment between portions of the material of the individual package after the first opening. It is not required that a resealable individual package is identical in configuration or strength before the first opening and after resealing. For example, resealing by use of an adhesive tab (49) will typically result in an adhesive attachment weaker than the adhesive attachment before the first opening.

The bag (11) and the flange (12), which are typically comprised by a human waste management device, may or may not be folded when the device is provided in the individual package. If the bag (11) is not folded the individual package will typically have to provide more volume than for a folded bag (11). Thus, preferably at least the bag (11) is folded. A preferred folding pattern for the bag (11) is disclosed in patent application No. PCT/US98/13298 ("Applicator for a faecal management device") and shown in Figures 2 and 3.

A preferred bag (11) for an urinary management device (100) has a T shape as shown in Figure 5. A preferred folding pattern for such a bag (11) is achieved by first folding about the two axes a and then folding about axis b. Any other folding pattern involving an additional folding axis parallel to axis b or additional axes, whether parallel to axes a and b or not, and all directions of folding are within the scope of the present invention.

One particularly preferred embodiment of the present invention is depicted in Figure 2. Therein, the individual package is provided from a single sheet of plastic wrapper (48) which is folded around a faecal management device (10) for a baby. The faecal management device (10) is provided with an applicator (40). Preferred applicators are disclosed in patent application PCT/US98/13298. While any pattern of folding of such a wrapper (48) is within the scope of the present invention, the preferred folding is C-folding as depicted in Figures 2 and 3. Such a C-folded wrapper (48) is folded about two separated lines. These lines define the direction which is referred to as the "transversal direction" with regard to the wrapper (48). The direction vertical to the direction of the folding line is referred to as the "longitudinal direction". Preferably, the folding lines are so chosen that portions of the wrapper (48) overlap as shown in Figure 3. To maintain such a folded arrangement preferably a tab (49) is provided. The tab (49) is preferably permanently attached to one portion of the wrapper (48) and releasably attached to another portion of the wrapper (48). Permanent attachment can be achieved by means such as permanent adhesive attachment or thermobonding, ultrasonic bonding, thermocrimping, cold crimping, adhesive. The tab (49) is also to be understood as permanently attached to the wrapper (48) if it is a continuous extension of the wrapper. If portions of the wrapper (48) overlap, the tab is preferably used to maintain such overlapping arrangement and preferably connects portions of the wrapper along a longitudinal line.

The C-folded wrapper (48) is preferably sealed along longitudinal lines close to the longitudinal ends of the wrapper (48). Such sealing renders the individual package more protective, in particular against contamination by dirt or dust. The sealing can be provided by any means known to the man skilled in the art, a preferred means being thermobonding. At least portions of the sealing should be easy to separate so as to not to obstruct the opening of the individual package. Thermobonding is used for the preferred embodiment as shown in Figure 2.

Such sealing may further be provided along a transversal line, preferably a transversal line in the portion of overlap of a C-fold wrapping configuration. Preferably, such transversal sealing line allows easy separation. Such transversal sealing line may be used in combination with other opening means or replace them. Such transversal sealing lines may be desirable for additional protection against dust, dirt or the like.

The tab (49) may comprise a means for initiating removal, e.g. an adhesive free gripping portion, which may inter alia be provided by folding a portion of the tab (49) onto itself.

The tab (49) is not only a preferred opening means, but also allows convenient resealing of the individual package, so that the individual package is suitable of the transport of a human waste management device (10) after use. Resealing of the device reduces and preferably prevents leakage of contained material and the escaping of malodour. It is thus a benefit of the present invention that the same individual package is suitable to contain the device before and after use.

Generally the wrapper (48) should be larger in at least one and preferably two dimensions than the device in its configuration for packaging. The configuration for packaging typically comprises a folded bag (11) and may comprise an applicator (40).

For a rectangular wrapper the length ratio of the two sides is preferably from 1:5 to 1:0.2, more preferably from 1:2 to 1:0.5 and most preferably from 1:1.5 to 1:0.66. The transversal folding lines are preferably so positioned that their distances as measured in the longitudinal direction to a first transversal end of the wrapper (48) expressed relative to the length of the wrapper (48) in the longitudinal direction are as follows: 15 % to 45%, preferably 20 % to 30 % for the first transversal folding line and 65 % to 85 %, preferably 70 % to 80 % for the second folding line. Preferably the portion of overlap measures at least 1 cm in the longitudinal direction. The size of the release sheet (47) is also to be adapted to the size of the wrapper (48).

In the preferred embodiment as depicted in Figure 2 the wrapper (48) is provided from a rectangular piece of plastic material measuring 150 mm x 230 mm. This size is appropriate for a faecal management device (10) for a baby comprising a bag in a truncated cone shape, which has a largest diameter of 18 cm and which is folded for packaging. The tab (49) in the preferred embodiment of Figure 2 has a size of 10 mm x 30 mm. The tab (49) is permanently attached in a central position on one of the transversal ends of the wrapper (48); this end is referred to as the tab end. The first folding line is positioned 60 mm away from the tab end, the second folding line is positioned 170 mm away from the tab end, leading to an overlap of the tab end over the other transversal end of the wrapper (48) of about 20 mm, as measured in the transversal direction.

This preferred embodiment further comprises as a release means a rectangular release sheet provided from a siliconised craft paper of 120 mm x 140 mm. One transversal end of the release sheet (47) is juxtaposed parallel to the tab end of the wrapper (48). The release sheet is attached to the plastic sheet by two adhesive stripes, following the transversal direction.

All sizes given above are for the preferred embodiment of the present invention depicted in Figure 2 and should be adapted for other embodiments. For example, a faecal management device (10) for an adult will typically require a larger wrapper (49). The size of the wrapper (48) or other individual package is also be chosen in view of the size of a human waste management device (10) containing faecal matter or urine, when it is intended also for the transport of the used device.

The wrapper (48) can be opened by pulling the tab (49). The tab (49) will then detach from the portion of the wrapper (48) to which it is releasably attached. The portion of the wrapper (48) adjacent to the tab end is referred to as "tab portion". Then the tab portion of the wrapper (48) is lifted and thus separated from the portion of the wrapper (48) which was overlaid, and thereby portions of the thermobonding on the longitudinal ends of the wrapper (48) are also detached. When the wrapper (48) is opened (as depicted in Figure 4) it comprises only one fold, providing a pocket, which is maintained by the remaining thermobonding along the longitudinal ends of the wrapper (48). For the preferred embodiment of the present invention which is depicted in Figures 2 to 4, for a faecal management device comprising an applicator, the applicator is oriented towards said pocket, whereas the flange (12) is oriented towards the release sheet (47) and thus in the direction of the opening of said pocket.

The embodiment of Figures 2 - 4 can be closed by use of the adhesive tab (49), which can be reattached to the portion of the wrapper (48) from where it was released or a nearby portion of the wrapper (48). Resealing the wrapper (48) by using the tab (49) does not lead to a reattachment of the thermobonded areas.

In another preferred embodiment of the present invention the device (10) is oriented so, that the flange (12) is contained in said pocket and the device (10) can be conveniently handled by holding the applicator (40) when separating the device (10) and the wrapper (48).

In another preferred embodiment the present invention comprises a wrapper (48) which does not comprise a separate release sheet (47), but which has a release surface such as a siliconised surface. The wrapper (48) can then serve as a release means for adhesive portions of the flange (12) and also as an individual package. Such a wrapper (48) may comprise portions which serve as an opening means. Resealing of such portions may be achieved by bringing the wrapper (48) in contact with adhesive portions of the human waste management device.

Another preferred embodiment of the present invention is an individual package comprising a pouch. Such a pouch may be provided by one continuous piece of material or by separate pieces of material. For example, a pouch can be made from one rectangular sheet of plastic material which is folded about one line and which is permanently sealed, e.g. by thermobonding, along two lines which are perpendicular to the folding line. The pouch will then have an orifice which is disposed between the two sealing lines. This orifice can be provided with any of the resealabte opening means described above. The orifice may also be provided with a flap, which may be provided with an opening means.

## Claims

1. A human waste management device comprising an individual package, characterised in that said individual package comprises a resealable opening means.

2. Human waste management device according to Claim 1, characterised in that said opening means comprises an adhesive.

3. Human waste management device according to any one of the preceding claims, characterised in that said resealable opening means comprises a tab (49).

4. Human waste management device according to any one of the preceding claims, characterised in that said individual package comprises a release means (47).

5. Human waste management device according to any one of the preceding claims, characterised in that said individual package comprises a one-part wrapper (48).

6. Human waste management device according to Claim 5, characterised in that said one-part wrapper comprises portions which overlap.

7. Human waste management device according to Claim 5, characterised in that said one-part wrapper (48) comprises a C-fold.

8. Human waste management device according to any one of the preceding claims, characterised in that said individual package further comprises an applicator (40).

9. Human waste management device according to any one of the preceding claims, characterised in that said human waste management device is folded.

10. Use of an individual package for the transport of a human waste management device containing human waste.
